(19) Europäisches Patentamt / European Patent Office / Office européen des brevets

(11) **EP 2 189 111 A1**

(12) **EUROPEAN PATENT APPLICATION**

(43) Date of publication:
**26.05.2010 Bulletin 2010/21**

(51) Int Cl.:
**A61B 5/0205** (2006.01)  **A61B 5/029** (2006.01)

(21) Application number: **08169615.5**

(22) Date of filing: **21.11.2008**

| | |
|---|---|
| (84) Designated Contracting States:<br>**AT BE BG CH CY CZ DE DK EE ES FI FR GB GR HR HU IE IS IT LI LT LU LV MC MT NL NO PL PT RO SE SI SK TR**<br>Designated Extension States:<br>**AL BA MK RS** | (72) Inventor: **Dr. Joeken, Stephan**<br>**79839, Lörrach (DE)** |
| (71) Applicant: **Pulsion Medical Systems AG**<br>**81829 München (DE)** | (74) Representative: **Ettmayr, Andreas et al**<br>**Kehl & Ettmayr**<br>**Patentanwälte**<br>**Friedrich-Herschel-Straße 9**<br>**81679 München (DE)** |

(54) **Apparatus and method for determining a physiologic parameter**

(57) An apparatus for determining physiologic parameters of a patient (6) comprises a pressure sensor adapted to provide readings of a blood pressure of said patient (6), which are stored as at least one pressure curve over time or a derivative thereof with respect to time, and evaluation means (4) adapted to determine, from said pressure curve or said derivative, at least one cardiac activity state variable representing cardiac activity over time and/or variation of cardiac activity over time, and to determine at least one cardiac preload state variable representing cardiac preload over time and/or variation of cardiac preload over time. The evaluation means (4) are further adapted to determine said physiologic parameter as a sum of a plurality of sum terms, at least one of which is a monotonous function of a cardiac activity state variable and at least another one of which is a monotonous function of a cardiac preload state variable.

Fig. 2

EP 2 189 111 A1

**Description**

The present invention relates to an apparatus for determining at least one physiologic parameter of a patient. In particular, the invention relates to an apparatus for determining at least one physiologic parameter of a patient which comprises a pressure sensor device adapted to provide readings of a blood pressure of said patient, storage means for storing said readings as at least one pressure curve over time or a derivative thereof with respect to time, and evaluation means adapted to determine, from said pressure curve or said derivative, at least one cardiac activity state variable representing cardiac activity over time and/ or variation of cardiac activity over time and said evaluation means further adapted to determine at least one cardiac preload state variable representing cardiac preload over time and/ or variation of cardiac preload over time.

Furthermore, the invention also relates to a method of determining at least one physiologic parameter of a patient reading in readings of a blood pressure of said patient, storing said readings as at least one pressure curve over time or a derivative thereof with respect to time, and determining, from said pressure curve or said derivative, at least one cardiac activity state variable representing cardiac activity over time and/ or variation of cardiac activity over time and at least one cardiac preload state variable representing cardiac preload over time and/ or variation of cardiac preload over time.

Many different techniques have been presented in the past to study the relation of stroke volume and cardiac preload of beings.

Frédéric Michard and Jean-Louis Teboul, "Predicting fluid responsiveness in ICU patients Chest 121(2002), 2000-2008 and DA Reuter et al., "Optimizing fluid therapy in mechanically ventilated patients after cardiac surgery by on-fine monitoring of left ventricular stroke volume variations. Comparison with aortic systolic pressure variations. " Br. J. Anaesth. 88 (2002), 124-126 disclose using the parameters stroke volume variation (SVV) and pulse pressure variation (PPV) for determining volume-responsiveness of a patient. However, this approach is limited to controlled mechanically ventilated patient and cannot be applied to spontaneously breathing patients.

For assessing volume-responsiveness of a spontaneously breathing patient, Monnet, X., Rienzo, M., Osman. D., Anguel, N., Richard, C., Pinsky, M. R. & Teboul, J. (2006) "Passive leg raising predicts fluid responsiveness in the critically ill", Critical care medicine, 34, 1402-7 suggests raising the legs of the patient in order to vary preload. However, depending on the particular circumstances, such as injuries of the monitored patient, mechanically raising the patient's legs in a defined manner may be difficult or virtually impossible.

Further, US 5769082 discloses a method of analyzing changes in continuously measured hemodynamic parameters in response to a set of predetermined changes in airway pressure or tidal volume. The method is generally called "respiratory systolic variation test" (RSVT). The analysis of the change in the hemodynamic parameter in response to such airway pressure maneuver serves as a non-invasive or minimally invasive method of assessing the cardiovascular status, particularly the volume responsiveness of the patient.

US 2004/0249297 relates to an apparatus for determining cardiovascular parameters, in particular for the continuous determination of the parameters that characterize a patient's left ventricular pumping action, and an apparatus for the continuous determination of the cardiac volume responsiveness indicator. However, it is a precondition to know a numerical value of a patient's left ventricular pumping action for further determination of cardiac volume responsiveness. Further, a third sensor for measuring, e.g. a strain-gauge, is required.

EP 1884189 describes a technique of determining a parameter usable to characterize volume responsiveness. Other physiologic parameters (such as cardiac output or tidal volume) may also (or alternatively) be determined. A typical graph of cardiac output, according to the Frank-Starling-law of the heart, depending on preload (or blood volume) is illustrated. Depending on the local slope of the graph, additional volume may greatly increase cardiac output or not increase cardiac output at all. This will further help to assess volume responsiveness in clinical practice. Nevertheless, it is not possible to determine to what extent the stroke volume will increase.

It is therefore an object of the present invention to provide an apparatus of the type initially mentioned allowing to correctly account for the influence of the present breathing state of the patient. Further, it is an object of the present invention to allow applying an apparatus of the type initially mentioned for mechanically ventilated patients and spontaneously breathing patients alike. Under one aspect, it is a particular object of the invention to provide an apparatus of the type initially mentioned, wherein the determined physiological parameter improves assessment of volume-responsiveness of the patient, regardless whether the patient is mechanically ventilated or spontaneously breathing.

Under one aspect of the present invention, the above objects are achieved by an apparatus according to claim 1. Advantageous embodiments of the present inventions can be configured according to any of claims 2-17.

Likewise, it is an object of the present invention to provide a method of the type initially mentioned allowing to correctly account for the influence of the present breathing state of the patient. Further, it is an object of the present invention to allow applying a method of the type initially mentioned for mechanically ventilated patients and spontaneously breathing patients alike. Under one aspect, it is a particular object of the invention to provide a method of the type initially mentioned, wherein the determined physiological parameter improves assessment of volume-responsiveness of the patient, regardless whether the patient is mechanically ventilated or spontaneously breathing.

Under one aspect of the present invention, the above objects are achieved by a method according to claim 18.

The present invention is applicable to spontaneously breathing living beings as well as to patients with assisted breathing or fully controlled ventilated patients. Moreover, if volume responsiveness is to be determined, no additional effort is necessary (such as leg raising manoeuvre, fluid or drug delivery), so that fluid responsiveness can be determined in clinical practice by making use of the approach described herein.

Further, neither surgical procedures nor a manipulation of patients is required. Besides, the present invention does not require any preceding determination of a stroke volume and/ or a stroke volume variation. In particular, the present invention allows a differentiated determination of the volume-responsiveness between the responsive and the non-responsive circulatory system states and especially between the different levels of responsiveness.

Corresponding to the arterial pressure, the lunge volume and the respiration pressure in the lung are varying. There are different types of parameters to characterize the state of lung, like the central venous pressure, the tidal volume and further respiration pressure of the respirator (including respiratory mask, tubus and conductive tubes), (thoracic- and bio-) measuring of impedance, intrathoracic pressures, etc.. At least two variables of state (Z1, Z2, ...) have to be generated from the cardiac variations (e.g. arterial pressure) and further from a parameter (e.g. the central venous pressure or the arterial pressure) which is affected by shifts in cardiac preload or by respiration respectively. The sum and/ or the difference of the above-mentioned variables of state represent the fluid responsiveness index.

Further, the variables of state are adapted for characterizing the cardiac and the respiratory activity and consequently the changes in preload, especially considering the effective forces, energies and powers. The sum/ difference of the variables of state is an indicator of the volume-responsiveness. Further, the variables of state can take into account the different characteristics of the vascular-and/ or thoracic systems.

The method can be used without preliminary calibration, if the parameters, which are specified by cardiac variation and respiration respectively, are scaled adequately. Complementary to the fluid responsiveness index, the absolute measuring of the stroke volume may be performed after calibration.

Further, the measured signals do not have to originate from intravascular pressure measurements. The measured signals for cardiac characterization and for changes in cardiac preload (e.g. with a respiratory activity) may be of the same kind. Further, the present invention allows a continuous determination of the stroke volume and the cardiac output after calibration of the relative volume-responsiveness. The value of cardiac output results from a multiplication of heart rate and stroke volume To determine the stroke volume and the cardiac output, the parameters of weight, height, surface of body of a patient may serve for an adaptation instead of using any calibration. Further, at least the first derivative can be used instead of the measured numerical value. Concerning the determination of the cardiac output, the blood flow is directly proportional to the calculus $dP/dt$ of pressure specified in equation 5 below.

Generally, any of the embodiments described or options mentioned herein may be particularly advantageous depending on the actual conditions of application. Further, features of one embodiment may be combined with features of another embodiment as well as features known per se from the prior art as far as technically possible and unless indicated otherwise.

The invention will now be described in more detail. The accompanying drawings, which are schematic illustrations, serve for a better understanding of the features of the present invention.

Therein

Fig.1 is a diagram illustrating the concept of volume-responsiveness by showing a typical graph of cardiac output over preload,

Fig. 2 illustrates the general setup of an apparatus according to a first embodiment of the present invention,

Fig. 3 illustrates the general setup of an apparatus according to a second embodiment of the present invention,

Fig. 4a shows a typical plot of arterial pressure readings varying with the cycle of breathing,

Fig. 4b shows a typical plot of central venous pressure readings varying with the cycle of breathing,

Fig. 5a shows a typical power spectrum based on readings of arterial pressure in logarithmic scaling,

Fig. 5b shows a typical power spectrum based on readings of arterial pressure in linear scaling,

Fig. 6a shows a typical power spectrum based on readings of central venous pressure in logarithmic scaling and,

Fig. 6b shows a typical power spectrum based on readings of central venous pressure in linear scaling.

In the drawings, the same reference numerals have been used for corresponding features.

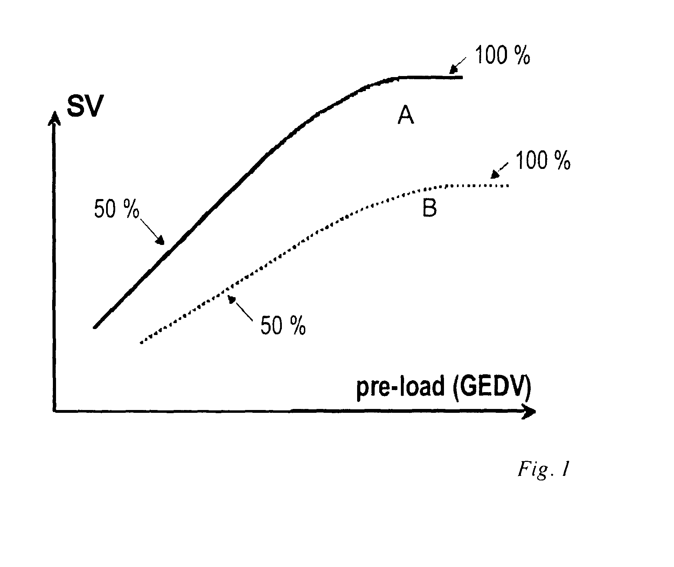

Fig. 1 shows a diagram illustrating the concept of volume-responsiveness by showing a typical graph of stroke volume (SV) over preload (or blood volume). The relation between stroke volume and blood volume is illustrated for two beings A (solid line) and B (dashed line) in Fig.1, according to the Frank-Starling-law of the heart. The graph varies from patient to patient (and depends on the individual patient's current condition). Thus, one value of the stroke volume can correspond with two different values of preload (and vice versa), depending on the patient. Depending on the local slope, additional fluid volume may greatly increase (left part of the diagram) or not increase stroke volume (nearly horizontal line in the right part of the diagram). As the actual course of the curve schematically shown in Fig. 1 is not known beforehand for a specific patient in a specific condition, acquiring parameters helping to assess volume responsiveness can be crucial in clinical practice. The higher the stroke volume is according to the Frank-Starling-law of the heart, the higher the sensibility of values will be. In other words: If the stroke volume is high, small changes of stroke volume correspond to a considerable change of preload Above a certain stroke volume, the derivative dpreload/dSV greatly increases.

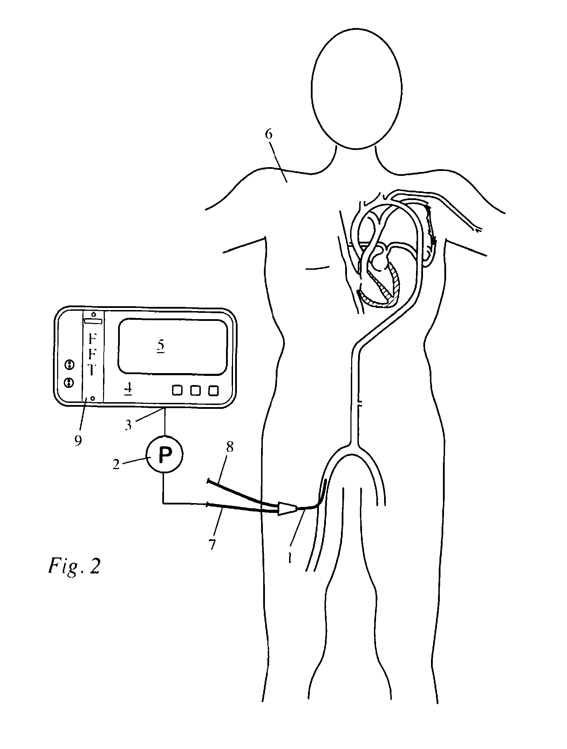

Fig. 2 shows the general setup of an apparatus of the present invention. An arterial catheter 1 is equipped with a pressure sensor for measuring arterial blood pressure. The pressure sensor of the catheter 1 is connected, via a pressure transducer 2, to an input channel 3 of a patient monitoring apparatus 4. Beside a proximal port 7 used to acquire the pressure signal, the catheter 1 may comprise one or more other proximal ports 8 to perform additional functions, such as blood temperature measurements or the like. The patient monitoring apparatus 4 is programmed to determine various hemodynamic parameters as described below, and to display the determined parameters (as numeric values, graphically or both) on the display 5. In addition, the determined parameters may be stored at a recording medium and/or printed. For this purpose, the patient monitoring apparatus 4 may comprise various interface ports for connecting peripheral equipment.

The first embodiment described requires a single arterial pressure sensor only. Though the sensor is shown to be invasive, a non-invasive pressure sensor may be implemented instead.

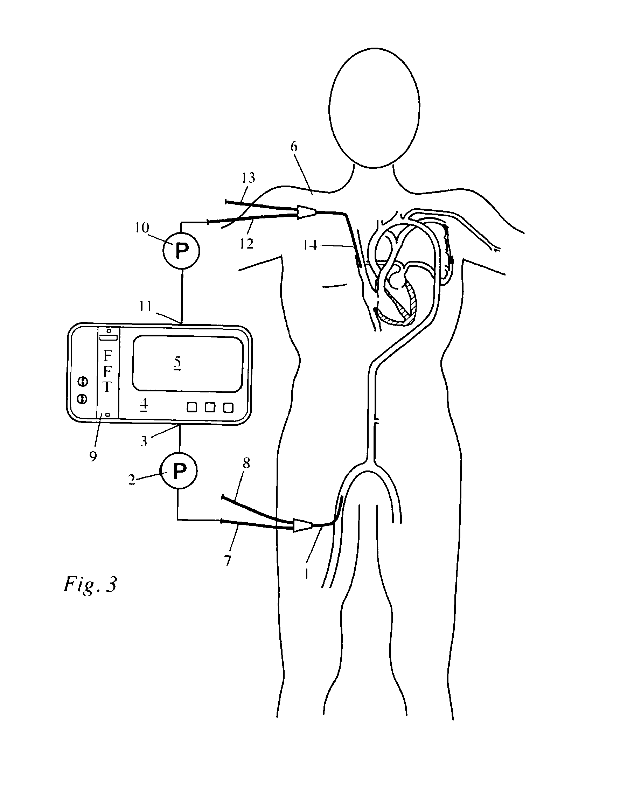

Fig. 3 further shows the general setup of an apparatus according to the second embodiment, wherein two pressure sensors are used. In addition to the arterial pressure measured as described in connection with the above first embodiment, a central venous pressure CVP is measured using a pressure sensor in a central venous catheter 14. The pressure sensor of the central venous catheter 14 is connected, via a pressure transducer 10, to a second input channel 11 of the patient monitoring apparatus 4. Beside a proximal port 12 used to acquire the pressure signal, the catheter 14 may comprise one or more other proximal ports 13 to perform additional functions, such as blood temperature measurements, injections or the like. Instead of the central venous catheter 14 a pulmonary artery catheter (not shown) may be used to provide readings of a pulmonary artery pressure. Generally, various measurement sites are suitable for providing first and second blood pressure readings. Best performance of the system can be achieved with two invasive pressure sensors, as depicted in Fig. 3.

As described above, various implementations of the invasive pressure sensors can be particularly advantageous. Pressure can either be transmitted hydraulically to a proximal catheter port and measured by an external sensor or may be measured directly on-site using a sensor installed at or near the catheter tip. Capacitative sensors, piezo sensors or optical pressure sensors (e.g. based on Fabry-Perot interferometer) may be used.

In a preferred embodiment of the present invention, at least one pressure sensor may also be non-invasive, as mentioned in connection with the first embodiment described above.

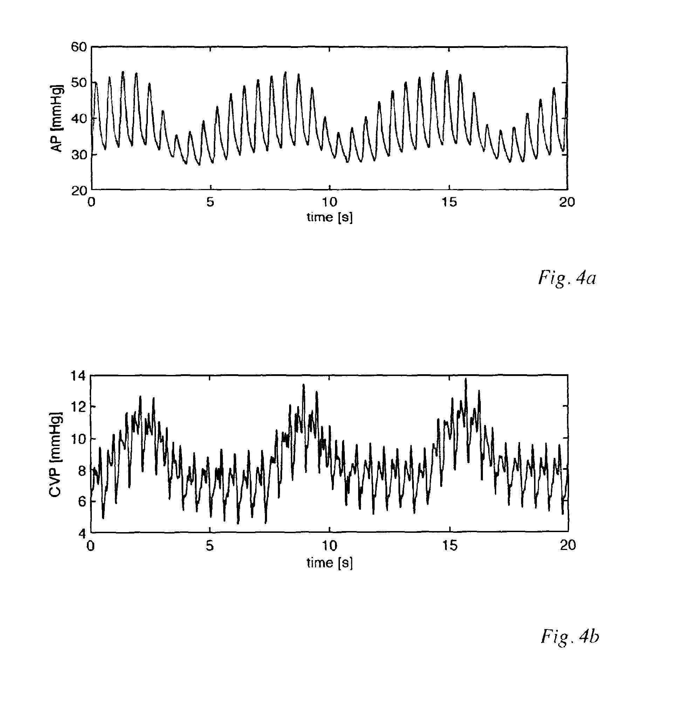

As explained above, cardiac, vasculary and pulmonary volumes interact with each other in the patient 6. In particular, cardiac preload is affected by the volume occupied by respiration (either spontaneous or ventilated breathing). Due to recurrent respiratory cycles modulation of blood pressure and blood flow take place. Fig. 4a shows this modulation in a typical plot of arterial pressure readings over time varying with the cycle of breathing Such a modulation can also be observed for central venous pressure or stroke volume, according to Fig. 4b.

The patient monitoring apparatus 4 temporarily stores the blood pressure readings read in through the input channel 3 as a pressure curve p(t) over time. As heart rate and breathing cycle differ in frequency (f), the respiratory effect on the pressure curve can be separated from the heart activity. The patient monitoring apparatus 4 thus determines breathing cycle and heart rate from the pressure signal.

In general, the determination of the volume-responsiveness leads to a specific therapy control of beings and especially of human beings. It is further relevant to come to a decision, whether to supply volume or the derivate volume to a patient. These manipulations of volume, for instance with the supply of physiologic saline solution, crystalloid or colloid liquid (e.g. HES), blood bottles or other fluid, are performed in accordance with diversifying clinical edge conditions, like emergency room, operating room, during surgical procedures, etc. Next to the artificially ventilation a patient could also ventilate non-artificial. The arterial and venous pressure [in mm Hg] is shown over a time of 20 seconds.

The methods of the current clinical practice nowadays are limited to the use of total controlled ventilated patients. The basis for the methods is a gain of lung volume as a result of respiration pressure of the respirator and a synchronous loss of the diastolic volume of the heart, because the lunge volume and the diastolic volume do merely have the same thoracic volume provided. As depicted in Figs. 4a, b, during the mechanical ventilation, the stroke volume deceases, when inhaling air into the lunge. Consequently, the arterial pulse pressure and the stroke volume are varying during a circle of breathing (Fig. 4a).

The corresponding modulation can also be observed for central venous pressure or stroke volume (Fig. 4b).

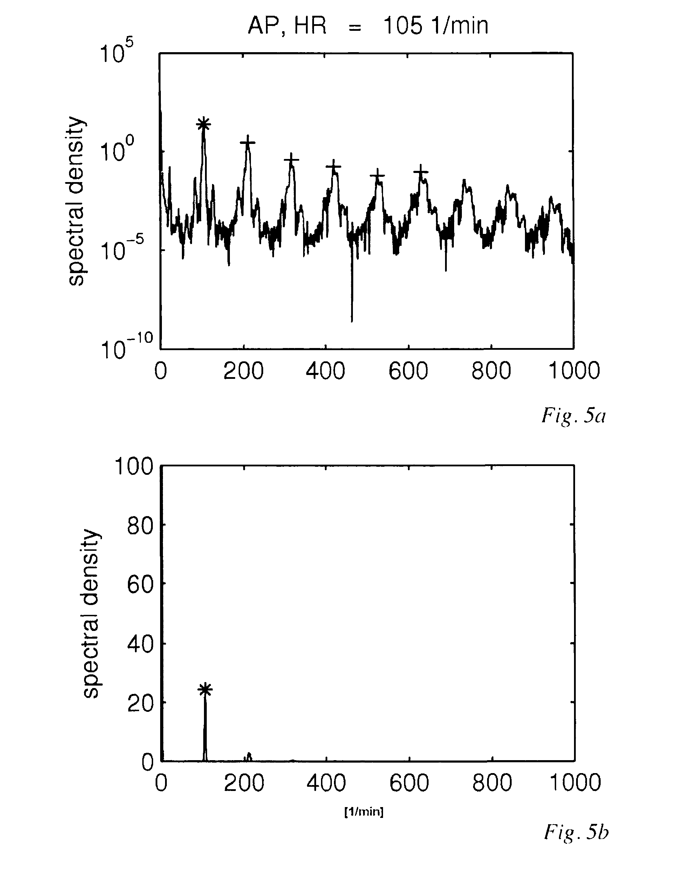

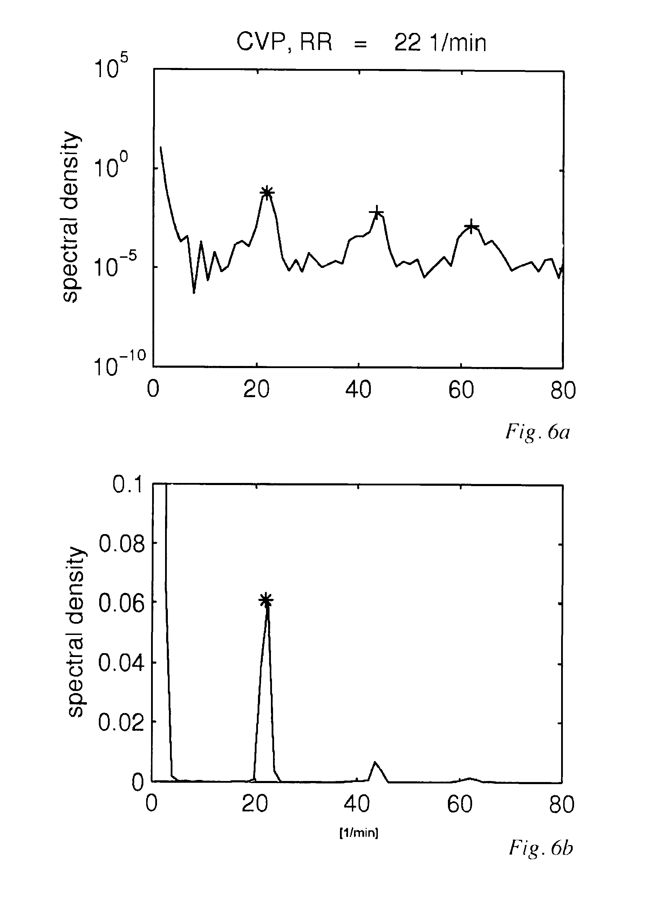

Figs. 5a, b show a typical power spectrum based on readings of arterial pressure and a typical power spectrum based on readings of central venous pressure with a heart rate of 105 beats per minute in logarithmic and linear scaling, respectively. Figs. 6a, b further show a typical power spectrum based on readings of central venous pressure with 22 breaths per minute in logarithmic and linear scaling, respectively.

The pressure $P_A$ is continuously measured in the aorta or in an central artery. The resultant medium blood pressure MAD and its variance $\sigma_A^2$ is further calculated. Besides, an intrathoracic or a central venous pressure CVP is measured. The resultant variance $\sigma_{CVP}^2$ is further calculated.

A cardiac state is characterized by the sum $Z_k = a \cdot MAD + b \cdot \sigma_A^2 + ...$ using MAD and $\sigma_A^2$. The letters a, b (and also c, ..., f) have optional positive or negative contents.

A respiratory state is characterized by the sum $Z_r = c \cdot \sigma_{CVP}^2 + ...$ using $\sigma_{CVP}^2$. Further summands therein could also have data from $P_A$ and resultant deviated variables.

A parameter is then developed from the sum and from the difference respectively, representing the relative cardiac output of the heart, i.e.

$$\text{relative cardiac output} = e \cdot Z_k - f \cdot Z_r - ... \qquad (1)$$

In a variation of the most simple approach, the influence of the pulmonary vascular system (e.g. compliance) and the height, the weight and the surface area of a patient may be eliminated using adequate scale. Thus, the relative cardiac output between different patients as well as over an elongated space of time inside of the patient's body and also compared with each other. Especially parameters which are adequate for characterizing the cardiac activity (e.g. MAD, $\sigma_A^2$) can be scaled by division.

The relative cardiac output shall further state which size the current stroke volume does have in contrast to the maximal achievable stroke volume. In order to reproduce the physiologic relations in an exact way, the relative cardiac output has to be limited and further the states or their sums, too.

A sigmoid-function $\alpha (Z)$ acts as a limitation to reproduce the physiologic relations:

$$\lim_{Z \to -\infty} \alpha (Z) = 0, \lim_{Z \to +\infty} \alpha (Z) = 1 \text{ and } d\alpha /dZ \geq 0 \text{ for all } Z.$$

Next to temporal average values, variances and further cumulates and moments respectively and also transformed parameters are relevant for characterizing the states. In particular, the patient monitoring apparatus 4 advantageously contains fast Fourier transformation means (FFT) 9 in order to perform a Fourier transformation on the stored pressure curve. The Fourier transform

$$P (\omega) = \int p(t) \cdot e^{(-i \cdot \omega \cdot t)} dt \qquad (2)$$

of a pressure as well as the power spectrum $Sp(\omega)$ are further relevant, i.e. the Fourier transform of the autocorrelation function, which is also determined by the FFT, are used to determine the contribution of each frequency $f = \omega / 2\pi$ for further evaluation. The power spectrum, i.e. the Fourier-transformator of the function of autocorrelation offers among others the possibility, to separate the portion of signals, which correspond to the heart activity with heart rate and the multiple of the heart rate ($2 \cdot HR, 3 \cdot HR, ...$) from the correlating respiratory rate and the multiple of the respiratory rate, too.

In particular, the patient monitoring apparatus 4 determines in the spectral density of the pressure signal the magnitudes for the respiration rate and higher harmonics thereof, which leads to the respiratory power spectrum. Likewise, the cardiac power spectrum is determined from the amplitudes in the spectral density at the heart rate and higher harmonics thereof.

Integration of the spectral densities over the whole frequency range permits determination of a respiratory power corresponding to respiration and a cardiac power corresponding to heart activity. However, integration over only part of the frequency range will in many cases lead to sufficient approximations or even improve the quality of the results:

While the integrals have to run over a suitable range, several frequencies may be suppressed to reduce or eliminate signal disturbances.

Concerning Figs. 5a, b the spectral powers to the heart rates (HR) and the multiple thereof will be gained from the power spectrum. The peak marked with $(*)$ is $S \cdot (2\pi \cdot HR) = S_P \cdot (2\pi \cdot HR)$. The peaks marked with $(+)$ are $S = (2\pi \cdot k \cdot HR)$, $S_P = (2\pi \cdot k \cdot HR)$ for $k \in \{2, 3, 4, 5, 6\}$. Further, the spectral powers to the respiratory rate (RR) and to the multiple thereof, i.e. $S \cdot (2\pi \cdot RR)$, $S \cdot (2\pi \cdot 2 \cdot RR)$, etc., the areas and breadth of the particular peaks, the slope at the basis of the spectrum and over the tips of the peaks and the particular spectrum for $\omega \rightarrow 0$ will be gained from the power spectra.

Thus, the afore-mentioned parameters of the states can be characterized as follows:

A cardiac state is described by an adequate sum $Z_k = a \cdot \sigma_A^2 + b \cdot \sigma_A^2 / MAD + c \cdot S \cdot (2\pi \cdot k \cdot HR) + ...$ The necessary components are $\sigma_A^2$ and $\sigma_A^2 / MAD$ and at least one component of spectrum $S \cdot (2\pi \cdot k \cdot HR) + ...$ with adequate $k \in \{0, 1, 2, 3, ...\}$.

A stroke volume modification, according to Figs. 6a, b is described in general by the adequate sum $Zr = d \cdot S \cdot (2\pi \cdot l \cdot RR) + ...$ The therefore necessary component is the component of spectrum $d \cdot S \cdot (2\pi \cdot l \cdot RR) + ...$ with adequate $l \in \{0, 1, 2, 3, ...\}$. Further summands therein could also have data from $P_A$ and resultant deviated variables. The peak marked with $(*)$ is $S \cdot (2\pi \cdot RR) = S_{CVP} \cdot (2\pi \cdot RR)$. The peaks marked with $(+)$ are $S \cdot (2\pi \cdot k \cdot RR) = S_{CVP} \cdot (2\pi \cdot l \cdot RR)$ for $l = 2, 3$.

The thus determined respiratory and cardiac power spectra values can now be used by the patient monitoring apparatus 4 to calculate the hemodynamic parameters of interest and display the determined parameters on the display 5.

Subsequently, a parameter is then developed from the sum and the difference respectively, representing the relative cardiac output of the heart, viz.

$$\text{relative cardiac output} = g \cdot \alpha(Z_k) - h \cdot \alpha(Z_r) - ... \qquad (3)$$

Thus, this results in an equation of the fluid responsiveness index FRI of the following form:

$$FRI = g \cdot \tanh(Z_k) - h \cdot \tanh(Z_r) + ... \qquad (4)$$

wherein $Z_k$ preferably is a function of $\sigma_A$, $S_p$, $S_{dp/dt}$, MAD and Zr preferably is a function of $\sigma_A$. $S_{CVP}$. $S_{dp/dt}$, CVP.

As mentioned before, Fig. 2, shows a general setup of an apparatus of the present invention, wherein an arterial catheter 1 is equipped with a pressure sensor for measuring arterial blood pressure. In contrast to Fig. 2, Fig. 3 shows the general setup of an apparatus according to the second embodiment, wherein two pressure sensors are used. The first sensor is for arterial pressure measuring and the second sensor for central venous pressure measuring.

The varying lunge volume and the respiration pressure in the lung affect both arterial pressure and central venous pressure, as depicted in Figs. 4a, b. There are different types of parameters to characterize the state of breathing, as mentioned above. At least two variables of state (Z1, Z2, ...) are generated from the cardiac variations (e.g. arterial pressure) and from a further parameter (e.g. the central venous pressure or the arterial pressure). The parameter is affected by shifts in caridiac preload or by respiration respectively. The fluid responsiveness index is then represented by the sum and/ or the difference of the above-mentioned variables of state.

Especially, the patient monitoring apparatus 4, according to Fig. 3, determines in the spectral density of the pressure signal the magnitudes for the respiration rate and higher harmonics thereof, which leads to the respiratory power spectrum. The cardiac power spectrum is determined from the amplitudes in the spectral density at the heart rate and higher harmonics thereof.

The patient monitoring apparatus 4 determines the breathing cycle from the central venous pressure signal, according to Fig. 3. Using the fast Fourier transformator 9, the patient monitoring apparatus 4 determines the spectral density, according to Figs. 6a, b. In the spectral density the magnitudes are determined for the respiration rate and higher harmonics thereof, which leads to the respiratory power spectrum and consequently to the the respiratory power, as already described above.

Finally, the ratio of respiration and cardiac power is provided as a measure of volume responsiveness as described above in connection with the first embodiment. The second embodiment leads to a more precise value of relative cardiac output as shown in equations 1 and 3. Further, a more precise value of the fluid responsiveness index as shown in equation 4 and the following equations can be achieved.

As an alternative or in addition to the determination of the fluid responsiveness index FRI a stroke volume reserve index SVRI may be determined, which is defined as SVRI = 1 - FRI.

**Claims**

1. Apparatus for determining at least one physiologic parameter of a patient (6), said apparatus comprising:

   - a pressure sensor device adapted to provide readings of a blood pressure of said patient (6)
   - storage means for storing said readings as at least one pressure curve over time or a derivative thereof with respect to time,
   - evaluation means (4) adapted to determine, from said pressure curve or said derivative, at least one cardiac activity state variable representing cardiac activity over time and/ or variation of cardiac activity over time and wherein said evaluation means (4) are further adapted to determine at least one cardiac preload state variable representing cardiac preload over time and/or variation of cardiac preload over time,

   **characterized in that** said evaluation means (4) are further adapted to

   - determine said physiologic parameter as a sum of a plurality of sum terms, wherein at least one of said sum terms is a monotonous function of one said cardiac activity state variable and at least one of said sum terms is a monotonous function of one said cardiac preload state variable.

2. Apparatus according to claim 1, wherein said variation of cardiac preload over time is a variation of cardiac preload resulting from patient's (6) inspiration and expiration detectable during patient's (6) spontaneous breathing status, mechanical ventilation status or assisted breathing status.

3. Apparatus according to any of the preceding claims, wherein said monotonous function of said cardiac activity state variable is a product of a real number and said cardiac activity state variable.

4. Apparatus according to any of the preceding claims, wherein said monotonous function of said cardiac activity state variable is a product of a real number and a normalizing function of said cardiac activity state variable.

5. Apparatus according to claim 4, wherein said normalizing function is a sigmoid function.

6. Apparatus according to any of the preceding claims, wherein said monotonous function of said cardiac preload state variable is a product of a real number and said cardiac preload state variable.

7. Apparatus according to any of claims 2-5, wherein said monotonous function of said cardiac preload state variable is a product of a real number and a normalizing function of said preload activity state variable.

8. Apparatus according to claim 7, wherein said normalizing function is a sigmoid function.

9. Apparatus according to any of the preceding claims, wherein said evaluation means (4) are further adapted to determine at least one said cardiac preload state variable from said pressure curve or said derivative.

10. Apparatus according to any of the preceding claims, further comprising an additional sensor device providing additional readings.

11. Apparatus according to claim 10, wherein said additional readings correlate with cardiac preload, wherein said storage means are further adapted to store a progression of said additional readings over time or a derivative thereof with respect to time, and wherein said evaluation means (4) are further adapted to determine at least one cardiac preload state variable from said progression or said derivative thereof.

12. Apparatus according to any of claims 10-11, wherein said additional readings correlate with at least one cardiac activity state variable.

13. Apparatus according to any of preceding claims, wherein said preload state variable is a central venous pressure, an arterial wedge pressure, a global end-diastolic volume, a pulmonary capillary wedge pressure or an arterial pressure.

14. Apparatus according to claim 10, wherein said additional sensor device is an additional pressure sensor device.

**15.** Apparatus according to claim 14, wherein said additional pressure sensor device is adapted to measure central venous pressure.

**16.** Apparatus according to any of the preceding claims, wherein said pressure sensor device is adapted to measure arterial pressure.

**17.** Apparatus according to any of the preceding claims, wherein said pressure sensor device is adapted for non-invasive measurement.

**18.** Apparatus according to any of the preceding claims, wherein said at least one physiologic parameter includes Stroke Volume Reserve or Fluid Responsiveness Index.

**19.** Method for determining at least one physiologic parameter of a patient (6), said method comprising:

- reading in readings of a blood pressure of said patient (6)
- storing said readings as at least one pressure curve over time or a derivative thereof with respect to time,
- determining, from said pressure curve or said derivative, at least one cardiac activity state variable representing cardiac activity over time and/ or variation of cardiac activity over time and
- determining at least one cardiac preload state variable representing cardiac preload over time and/ or variation of cardiac preload over time,

**characterized in that** said physiologic parameter is determined as a sum of a plurality of sum terms, wherein at least one of said sum terms is a monotonous function of one said cardiac activity state variable and at least one of said sum terms is a monotonous function of one said cardiac preload state variable.

*Fig. 1*

*Fig. 2*

*Fig. 3*

*Fig. 4a*

*Fig. 4b*

AP, HR = 105 1/min

*Fig. 5a*

[1/min]

*Fig. 5b*

CVP, RR = 22 1/min

*Fig. 6a*

[1/min]

*Fig. 6b*

Europäisches
Patentamt

European
Patent Office

Office européen
des brevets

## PARTIAL EUROPEAN SEARCH REPORT

**Application Number**

which under Rule 63 of the European Patent Convention
shall be considered, for the purposes of subsequent
proceedings, as the European search report

EP 08 16 9615

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | EP 1 884 189 A (PULSION MEDICAL SYS AG [DE]) 6 February 2008 (2008-02-06) * claims 1-14; figures 2-9 * | 1-3,6, 9-18 | INV. A61B5/0205 A61B5/029 |
| X | US 6 315 735 B1 (JOEKEN STEPHAN [DE] ET AL) 13 November 2001 (2001-11-13) * abstract; claims 1-26 * | 1-3,6, 9-16 | |
| X | US 2005/267379 A1 (PFEIFFER ULRICH J [DE] ET AL) 1 December 2005 (2005-12-01) * abstract; claims 1-19; figure 1 * | 1-3,6, 9-16 | |
| X,D | US 2004/249297 A1 (PFEIFFER ULRICH J [DE] ET AL) 9 December 2004 (2004-12-09) * abstract; claims 1-29; figures 1-5 * | 1-3,6, 9-16,18 | |
| X | WO 2006/020037 A (EDWARDS LIFESCIENCES CORP [US]; HATIB FERAS [BG]; ROTELIUK LUCHY [US]) 23 February 2006 (2006-02-23) * abstract; claims 1-18; figures 1-7 * * paragraph [0041] * | 1-9,13, 16,17 | |

-/--

**TECHNICAL FIELDS SEARCHED (IPC)**

A61B

### INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| Munich | 17 February 2009 | Apostol, Simona |

EPO FORM 1503 03.82 (P04E07)

Europäisches
Patentamt

European
Patent Office

Office européen
des brevets

**PARTIAL EUROPEAN SEARCH REPORT**

Application Number

EP 08 16 9615

| | DOCUMENTS CONSIDERED TO BE RELEVANT | | CLASSIFICATION OF THE APPLICATION (IPC) |
|---|---|---|---|
| Category | Citation of document with indication, where appropriate, of relevant passages | Relevant to claim | |
| A | COUDRAY ALICE ET AL: "Fluid responsiveness in spontaneously breathing patients: a review of indexes used in intensive care" CRITICAL CARE MEDICINE, WILLIAMS AND WILKINGS COMPANY, BALTIMORE, MA, US, vol. 33, no. 12, 1 December 2005 (2005-12-01), pages 2757-2762, XP009097105 ISSN: 0090-3493 * the whole document * | | |
| A | HOFER CHRISTOPH K ET AL: "Stroke volume and pulse pressure variation for prediction of fluid responsiveness in patients undergoing off-pump coronary artery bypass grafting." CHEST AUG 2005, vol. 128, no. 2, August 2005 (2005-08), pages 848-854, XP002515370 ISSN: 0012-3692 * the whole document * | | TECHNICAL FIELDS SEARCHED (IPC) |

EPO FORM 1503 03.82 (P04C10)

Europäisches
Patentamt

European
Patent Office

Office européen
des brevets

**INCOMPLETE SEARCH
SHEET C**

Application Number

EP 08 16 9615

Claim(s) searched completely:
       1-18

Claim(s) not searched:
       19

Reason for the limitation of the search (non-patentable invention(s)):

Claim 19 is related to a method for determining at least one physiologic
parameter of a patient.
The method comprises the step of reading in readings of a blood pressure
of said patient. As it becomes apparent from the description, this step
is performed by means of a pressure sensor which is inserted, in at least
one of the embodiments, in the central venous or an artery via a
catheter. Therefore, the reading step involves a surgical intervention by
means of which the method as a whole is considered to be a method for
treatment of the human or animal body by surgery according to the Article
53(c) EPC. The method of claim 19 is hence excluded from patentability.

**EP 2 189 111 A1**

## ANNEX TO THE EUROPEAN SEARCH REPORT
## ON EUROPEAN PATENT APPLICATION NO.

EP 08 16 9615

This annex lists the patent family members relating to the patent documents cited in the above-mentioned European search report.
The members are as contained in the European Patent Office EDP file on
The European Patent Office is in no way liable for these particulars which are merely given for the purpose of information.

17-02-2009

| Patent document cited in search report | | Publication date | Patent family member(s) | | Publication date |
|---|---|---|---|---|---|
| EP 1884189 | A | 06-02-2008 | JP 2008036433 A<br>US 2008033306 A1 | | 21-02-2008<br>07-02-2008 |
| US 6315735 | B1 | 13-11-2001 | NONE | | |
| US 2005267379 | A1 | 01-12-2005 | BR PI0501917 A<br>CN 1698534 A<br>DE 102004024334 A1<br>EP 1598005 A1<br>JP 2005329237 A<br>KR 20060046075 A | | 10-01-2006<br>23-11-2005<br>22-12-2005<br>23-11-2005<br>02-12-2005<br>17-05-2006 |
| US 2004249297 | A1 | 09-12-2004 | DE 10260762 A1<br>EP 1434141 A2<br>JP 3857684 B2<br>JP 2004202250 A | | 22-07-2004<br>30-06-2004<br>13-12-2006<br>22-07-2004 |
| WO 2006020037 | A | 23-02-2006 | CA 2570144 A1<br>EP 1773187 A1<br>JP 2008506472 T | | 23-02-2006<br>18-04-2007<br>06-03-2008 |

EPO FORM P0459

For more details about this annex : see Official Journal of the European Patent Office, No. 12/82

18

## REFERENCES CITED IN THE DESCRIPTION

**Patent documents cited in the description**

- US 5769082 A
- US 20040249297 A
- EP 1884189 A

**Non-patent literature cited in the description**

- **Frédéric Michard ; Jean-Louis Teboul.** Predicting fluid responsiveness in ICU patients. *Chest,* 2002, vol. 121, 2000-2008
- **DA Reuter et al.** Optimizing fluid therapy in mechanically ventilated patients after cardiac surgery by on-fine monitoring of left ventricular stroke volume variations. Comparison with aortic systolic pressure variations. *Br. J. Anaesth.,* 2002, vol. 88, 124-126
- **Monnet, X. ; Rienzo, M. ; Osman. D. ; Anguel, N., ; Richard, C. ; Pinsky, M. R. ; Teboul, J.** Passive leg raising predicts fluid responsiveness in the critically ill. *Critical care medicine,* 2006, vol. 34, 1402-7